# EUROPÄISCHE PATENTSCHRIFT

(11) **EP 1 378 511 B1**
(45) Veröffentlichungstag und Bekanntmachung des Hinweises auf die Patenterteilung: **12.12.2007**
(21) Anmeldenummer: 03014035.4
(22) Anmeldetag: 20.06.2003
(51) Int. Cl.: C07D 487/04

(54) **Verfahren zur kontinuierlichen Herstellung von Acetylendiharnstoff**
Process for the continous preparation of Acetylenediureine
Procédé pour la préparation continue d'Acetylendiuréine

(30) Priorität: 06.07.2002 DE 10230490
(43) Veröffentlichungstag der Anmeldung: 07.01.2004
(73) Patentinhaber: COMPO Gesellschaft mbH & Co. KG, 48157 Münster (DE)
(72) Erfinder: Franke, Dirk, Dr., 67134 Birkenheide (DE); Horchler, Klaus, Dr., 67117 Limburgerhof (DE); Czikkely, Vilmos, Dr., 68167 Mannheim (DE)
(74) Vertreter: Filbry, Eberhard

(56) Entgegenhaltungen:
- US-A- 2 803 564
- DATABASE CA [Online] CHEMICAL ABSTRACTS SERVICE, COLUMBUS, OHIO, US; SHIMIZU, TOSHIO: "Glycoluril as a slow release nitrogen fertilizer" retrieved from STN Database accession no. 108:36719 XP002250668 & SOIL SCIENCE AND PLANT NUTRITION (TOKYO, JAPAN) ( 1987 ), 33(2), 291-8 ,
- DATABASE WPI Section Ch, Week 200114 Derwent Publications Ltd., London, GB; Class C02, AN 2001-127343 XP002250669 & JP 2000 290281 A (MITSUI CHEM INC), 17. Oktober 2000 (2000-10-17)

## Beschreibung

Die Erfindung betrifft ein Verfahren zur kontinuierlichen Herstellung des Stickstoff-Langzeitdüngers Acetylendiharnstoff.

Düngemittel mit Langzeitwirkung haben gegenüber konventionellen mineralischen oder organischen Düngemitteln viele Vorteile. Sie bieten eine bedarfsgerechtere Anlieferung der Nährstoffe an die Pflanze und verbessern dadurch die Nährstoffausnutzung. Dies führt zu einer Verringerung von Nährstoffverlusten, wodurch die Umweltbelastung reduziert und die Effizienz der Düngung gesteigert werden. Außerdem erlauben sie es, Arbeitsgänge und Betriebsmittel und somit agrarwirtschaftliche Kosten einzusparen.

Eine Langzeitwirkung von Düngemitteln kann auf unterschiedlichen Wegen erreicht werden. Eine Möglichkeit besteht darin, in Wasser leichtlösliche granulierte Düngemittel mit einer wasserunlöslichen Hülle zu umgeben. Die Nährstofffreisetzung aus solchen umhüllten Düngemitteln erfolgt verzögert, da die Nährstoffe erst durch die Hüllschicht hindurch diffundieren müssen, bevor sie von den Wurzeln aufgenommen werden können. Eine andere Möglichkeit besteht darin, die Nährstoffe in Form von chemischen Verbindungen auszubringen, in denen sie zunächst nicht pflanzenverfügbar sind. Erst nach Ablauf eines vorgelagerten Freisetzungsschrittes, zum Beispiel einer chemischen Hydrolyse, einer enzymatischen Spaltung und/oder einer mikrobiellen Umsetzung; liegen die Nährstoffe in einer für die Pflanzen verwertbaren Form vor. Derartige Düngemittel werden auch als chemische Langzeitdünger bezeichnet.

Die Idee der chemischen Langzeitdünger stammt bereits aus dem 19. Jahrhundert. Damals regten Liebig in Deutschland und Murray in England an, Nährstoffe in Form schwerlöslicher Salze für die Pflanzenernährung einzusetzen.

Heute werden eine ganze Reihe von stickstoffhaltigen Substanzen hergestellt und als Langzeitdünger vermarktet. Die drei mit Abstand wichtigsten sind dabei die Kondensationsprodukte aus Harnstoff und Formaldehyd, lsobutyraldehyd bzw. Acetaldehyd.

Weitere in kleinerem Maßstab zur Verwendung als Düngemittel hergestellte Substanzen sind unter anderem Oxamid, Acetylendiharnstoff, Melamin, substituierte Triazone und der nachstehend gezeigte Acetylendiharnstoff.

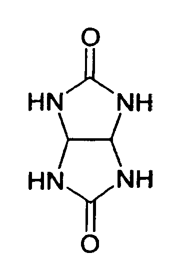

Von diesen bekannten Stickstoff-Langzeitdüngern ist Acetylendihamstoff von besonderem Interesse wegen seines vergleichsweise hohen Stickstoffgehalts, seiner sehr guten Pflanzenverträglichkeit sowie seines ausgezeichneten Wirkprofils. Seine Synthese aus Harnstoff und Glyoxal ist vielfältig beschrieben. L. Siemonsen berichtet in Ann. Chem. 333, (1904), Seiten 101 bis 111 über die Herstellung von Acetylendiharnstoff (Glycoluril) aus Glyoxal und Harnstoff unter Zusatz von Salzsäure.

In Chem. Ind. 1979, Seite 29 bis 30 wird die Herstellung von Acetylendiharnstoff (Glycoluril) durch Kondensation von Glyoxal und Harnstoff beschrieben. Dazu wurde fester Harnstoff in 40%-ige Glyoxallösung unter Rühren für 30 Minuten bei 70°C eingebracht. Der erhaltene ausgefällte Feststoff wurde abfiltriert, gewaschen und getrocknet.

T. Shimizu beschreibt in Soil. Sci. Plant. Nutr. 33, (1987, Seiten 291 bis 298) die Herstellung von Acetylendiharnstoff durch Umsetzung von Glyoxal mit Harnstoff in Gegenwart einer Säure als Katalysator. Zur Erhöhung der Ausbeuten wurden Konzentrationsreihen und Variationen des Katalysators durchgeführt. Zudem wurde nicht umgesetztes Glyoxal in die Umsetzung zurückgeführt. Die Umsetzung wurde dabei diskontinuierlich durchgeführt, wobei die Glyoxallösung tropfenweise in die Harnstofflösung dosiert wurde, um die Ausbeute zu maximieren. Die erhaltene Ausbeute betrug 87%. Als optimale Umsetzungsbedingungen werden eine Temperatur von 60 bis 80°C, eine Umsetzungszeit von 1,5 bis 3 Stunden und eine Konzentration an Salzsäure als Katalysator von 5 bis 10% angegeben. Nach 6-fachem Rückführen konnte die Gesamt-ausbeute auf 91% erhöht werden.

US 3,061,423 beschreibt den Einsatz von Acetylendiharnstoff (Glycoluril) als Düngemittel.

US 2,731,472 betrifft die Herstellung von heterozyklischen Verbindungen aus Glyoxal und Harnstoff unter saurer Katalyse.

JP 2001 097974 betrifft die Herstellung von Acetylendiharnstoff durch Umsetzung von Harnstoff mit Glyoxal in Gegenwart von Salzsäure als Katalysator. Das Molverhältnis von Harnstoff zu Glyoxal beträgt dabei 2,01 bis 2,30. Die Herstellung erfolgt diskontinuierlich, und ein Teil der Reaktionslösung, aus der Acetylendiharnstoff abgetrennt wurde, konnte in den Reaktor zurückgeführt werden. Vor dem Abfiltrieren des erhaltenen Acetylendiharnstoffs wurde mit Ammoniak neutralisiert.

JP 2000 264887 betrifft die Herstellung von Acetylendiharnstoff durch Umsetzung von Harnstoff und Glyoxal, wobei ebenfalls ein Molverhältnis von Harnstoff zu Glyoxal von 2,01 bis 2,3 eingestellt wird. Es wird mit gesättigter Harnstofflösung gearbeitet.

JP 2000 290281 betrifft die Herstellung von Acetylendiharnstoff durch Umsetzung von Harnstoff mit Glyoxal, wobei Harnstoff und Glyoxal kontinuierlich in eine gesättigte Suspension von Acetylenhamstoff in einem Molverhältnis von 2,01 bis 2,30 dosiert werden. Harnstoff und Glyoxal werden in wässriger Lösung in Gegenwart von kontinuierlich zugeführtem Säurekatalysator umgesetzt. Es wird angegeben, dass das Verfahren die kontinuierliche Herstellung von Acetylendiharnstoff erlaubt.

Nachteilig an den bekannten Verfahren ist aber entweder eine diskontinuierliche Reaktionsführung, welche bei der Herstellung großer Mengen ungünstig ist, der Einsatz eines größeren Harnstoffüberschusses, der letztendlich abgetrennt und verworfen werden muss oder der Anfall größerer Mengen an Nebenprodukten bzw. Mutterlauge, welche einer Entsorgung zugeführt werden müssen.

Aufgabe dieser Erfindung war es daher, ein kontinuierliches Syntheseverfahren zur Herstellung von Acetylendiharnstoff bereitzustellen, welches das Produkt mit hoher Ausbeute bei gleichzeitig geringem Anfall von Nebenprodukten sowie nur geringen Entsorgungsströmen liefert.

Die Aufgabe wird erfindungsgemäß gelöst durch ein Verfahren zur kontinuierlichen Herstellung von Acetylendiharnstoff durch Umsetzung von Glyoxal mit Harnstoff in Gegenwart von Mineralsäuren, **dadurch gekennzeichnet, dass** die Umsetzung in einer Reaktorkaskade durchgeführt wird, bestehend aus mindestens zwei Reaktoren, die jeweils als Rührkessel ausgeführt sind, wobei in mindestens einem Reaktor das Glyoxal, der Harnstoff und die Mineralsäure kontinuierlich eingetragen werden und aus mindestens einem Reaktor zusätzlich Wasser als Dampf ausgetragen wird und aus einem Reaktor eine Suspension von Acetylendiharnstoff in Mutterlauge ausgetragen wird, wobei der Acetylendihamstoff von der Mutterlauge mechanisch abgetrennt wird und die verbleibende Mutterlauge ganz oder teilweise in die Reaktor kaskade zurückgeführt wird.

Dabei werden als Reaktor oder Reaktoren Rührkessel eingesetzt. Die Reaktionspartner Glyoxal und Harnstoff werden kontinuierlich im Reaktor, insbesondere Rührkessel, zusammengeführt. Parallel wird als Katalysator eine Mineralsäure, vorzugsweise Salzsäure oder Schwefelsäure, insbesondere Schwefelsäure, dosiert. Der Harnstoff liegt dabei vorzugsweise im Überschuss gegenüber Glyoxal vor.

Im erfindungsgemäßen Verfahren wird eine Reaktorkaskade aus mindestens zwei Rührkesseln eingesetzt, die hintereinander geschaltet sind. Dies erlaubt die Einstellung einer hohen Verweilzeit und einer engen Verweilzeitverteilung. Die Gesamtverweilzeit in den Reaktoren beträgt vorzugsweise 1 bis 24 Stunden, besonders bevorzugt 2 bis 8 Stunden. Eine Verweilzeit von mehr als 3 Stunden ist dabei vorteilhaft.

Die Reaktorkaskade ist aus 2 bis 6 Rührkesseln, besonders bevorzugt 2 bis 3 Rührkesseln aufgebaut.

Die Umsetzung wird vorzugsweise bei einer Temperatur im Bereich von 50 bis 90°C, besonders bevorzugt im Bereich von 60 bis 80°C und einem Druck im Bereich von 0,05 bis 1 bar, besonders bevorzugt 0,1 bis 0,4 bar durchgeführt.

Der Acetylenhamstoff wird von der Mutterlauge mechanisch abgetrennt. Hierzu wird vorzugsweise ein Filtrationsaggregat wie ein Bandfilter eingesetzt. Nach der kontinuierlichen Abtrennung des gefällten Feststoffes wird die abgetrennte Mutterlauge zurück in den ersten Reaktorkessel geführt. Daher sind nur die als Katalysator eingesetzte Säure, insbesondere Schwefelsäure, und der Harnstoffüberschuss, die durch am Produkt anhaftende Restfeuchte verloren gehen, durch die frischen Lösungen zu ersetzen. Gegenüber der herkömmlichen kontinuierlichen Fahrweise reduziert sich der Bedarf an Katalysatorsäure und Harnstoff erheblich.

Bevorzugt wird der auf dem Filtrationsaggregat erhaltene Filterkuchen durch Behandlung mit neutralisierter Mutterlauge neutralisiert. Vorzugsweise erfolgt die Neutralisation des feuchten Filterkuchens direkt auf dem Filtrationsaggregat, indem die saure Mutterlauge abgetrennt, neutralisiert und zurück auf das Filtrationsaggregat gegeben wird. Vorzugsweise wird hierbei ein Bandfilter eingesetzt. Der abgetrennte Acetylendiharnstoff kann in einem Spinflash-Trockner getrocknet werden.

Erfindungsgemäß wird die verbleibende Mutterlauge teilweise oder vorzugsweise ganz in den (ersten) Reaktor zurückgeführt.

Hierbei war zu erwarten, dass sich unerwünschte Nebenprodukte allmählich aufkonzentrieren und letztlich entweder ins Produkt gelangen und dieses verunreinigen oder doch ausgeschleust und einer Entsorgung zugeführt werden müssen. Es wurde jedoch gefunden, dass beim erfindungsgemäßen Verfahren dies nicht der Fall ist, vielmehr die Nebenprodukte zum gewünschten Produkt weiterreagieren und zu einer gesteigerten Produktausbeute beitragen.

Das entstehende Reaktionswasser sowie ein Teil des mit den Edukten mitgeführten Wassers werden vorzugsweise in der Reaktion abgedampft. Die Abwasserströme reduzieren sich drastisch, sie beschränken sich lediglich auf die Restfeuchte. Werden die Bestandteile der Mutterlauge neutralisiert und als Nebenkomponente im Endprodukt akzeptiert, fallen nahezu keine Abwasserströme an, die Ammonium-Verbindungen enthalten. Die Abtrennung läßt sich besonders gut auf einem Bandfilter durchführen. Wird die Restfeuchte aus dem Kuchen entzogen, neutralisiert und wieder zurückgeführt, ist kein weiteres Aggregat zur Neutralisation notwendig.

Die Erfindung wird durch die nachstehenden Beispiele näher erläutert.

### Beispiele

### Beispiel 1:

In einer Kaskade, bestehend aus zwei gerührten Kesseln von jeweils circa 5 Liter Inhalt, werden kontinuierlich 933 g/h Harnstoff, 93 g/h Schwefelsäure und 1085 g/h (40,3%ig) Glyoxal zugegeben. Dabei werden je Kessel 359 g/h Wasser kontinuierlich abgedampft. Das Abdampfen wird so geregelt, dass kein zusätzliches Wasser in den Reaktor eingetragen werden muß. Die Reaktionstemperatur beträgt 65°C, der Druck circa 200 mbar. Die aus dem zweiten Kessel ausgeschleuste Suspension wird abgetrennt, und das Mutterfiltrat von circa 1200 g/h wird mit den Edukten zurück in den ersten Kessel gegeben. Die Ausbeute liegt bei > 97%. Außer der am Kuchen anhaftenden Restfeuchte von circa 15% wird kein Mutterfiltrat aus der Reaktion ausgeschleust.

### Vergleichsbeispiel 1

In einer Kaskade aus zwei Kesseln von jeweils circa 5 Liter Inhalt, werden kontinuierlich 460,5 g/h Wasser, 1492 g/h Harnstoff. 444 g/h Schwefelsäure sowie 1085 g/h Glyoxal (40,3%ig) zugefahren. Die Reaktionstemperatur beträgt 65°C. Die Ausbeute beträgt 90,5%, es fallen zusätzlich zur Restfeuchte von circa 15% über 2000 g/h Mutterlauge an, die entsorgt werden müssen.

## Patentansprüche

1. Verfahren zur kontinuierlichen Herstellung von Acetylendiharnstoff durch Umsetzung von Glyoxal mit Harnstoff in Gegenwart von Mineralsäuren, **dadurch gekennzeichnet, dass** die Umsetzung in einer Reaktorkaskade durchgeführt wird, bestehend aus mindestens zwei Reaktoren, die jeweils als Rührkessel ausgeführt sind, wobei in mindestens einem Reaktor das Glyoxal, der Harnstoff und die Mineralsäure kontinuierlich eingetragen werden und aus mindestens einem Reaktor zusätzlich Wasser als Dampf ausgetragen wird und aus einem Reaktor eine Suspension von Acetylendiharnstoff in Mutterlauge ausgetragen wird, wobei der Acetylendiharnstoff von der Mutterlauge mechanisch abgetrennt wird und die verbleibende Mutterlauge ganz oder teilweise in die Reaktor kaskade zurückgeführt wird.

2. Verfahren nach Anspruch 1, **dadurch gekennzeichnet, dass** die Umsetzung bei einer Temperatur im Bereich von 50 bis 90°C und einem Druck im Bereich von 0,05 bis 1 bar durchgeführt wird.

3. Verfahren nach einem der Ansprüche 1 bis 2, **dadurch gekennzeichnet, dass** die Gesamtverweilzeit in den Reaktoren 1 bis 24 Stunden beträgt.

4. Verfahren nach einem der Ansprüche 1 bis 3, **dadurch gekennzeichnet, dass** im Reaktor ein Überschuß an Harnstoff gegenüber Glyoxal vorliegt.

5. Verfahren nach einem der Ansprüche 1 bis 4, **dadurch gekennzeichnet, dass** zur mechanischen Abtrennung des Acetylendiharnstoffs von der Mutterlauge ein Filtrationsaggregat eingesetzt wird.

6. Verfahren nach Anspruch 5, **dadurch gekennzeichnet, dass** auf dem Filtrationsaggregat der erhaltene Filterkuchen durch Behandlung mit neutralisierter Mutterlauge neutralisiert wird.

7. Verfahren nach einem der Ansprüche 1 bis 6, **dadurch gekennzeichnet, dass** der abgetrennte Acetylendiharnstoff in einem Spinflash-Trockner getrocknet wird.

## Claims

1. A method of continuous production of acetylene diurea by reacting glyoxal with urea in the presence of mineral acids, **characterised in that** the reaction is carried out in a reactor cascade comprising at least two reactors which are each in the form of a stirring vessel, wherein the glyoxal, the urea and the mineral acid are continuously introduced in at least one reactor and water is additionally discharged in the form of vapour from at least one reactor and a suspension of acetylene diurea in mother liquor is discharged from a reactor, wherein the acetylene diurea is mechanically separated from the mother liquor and the remaining mother liquor is entirely or partially recycled into the reactor cascade.

2. A method according to claim 1 **characterised in that** the reaction is carried out at a temperature in the range of 50 to 90°C and at a pressure in the range of 0,05 to 1 bar.

3. A method according to one of claims 1 and 2 **characterised in that** the total residence time in the reactors is 1 to 24 hours.

4. A, method according to one of claims 1 to 3 **characterised in that** an excess of urea in relation to glyoxal is present in the reactor.

5. A method according to one of claims 1 to 4 **characterised in that** a filtration assembly is used for mechanical separation of the acetylene diurea from the mother liquor.

6. A method according to claim 5 **characterised in that** the filter cake obtained is neutralised on the filtration assembly by treatment with neutralised mother liquor.

7. A method according to one of claims 1 to 6 **characterised in that** the separated acetylene diurea is dried in a spin flash drier.

## Revendications

1. Procédé de préparation en continu d'acétylènediurée par réaction du glyoxal sur de l'urée en présence d'acides minéraux, **caractérisé en ce que** l'on effectue la réaction dans une cascade de réacteurs constituée d'au moins deux réacteurs qui sont réalisés respectivement en chaudron de brassage, le glyoxal, l'urée et l'acide minéral étant introduits en continu dans au moins un réacteur, et de l'eau supplémentaire étant sortie sous forme de vapeur d'au moins un réacteur et une suspension d'acétylènediurée dans de la liqueur mère étant sortie d'un réacteur, l'acétylènediurée étant séparée mécaniquement de la liqueur mère et la liqueur mère restante étant retournée en tout ou partie à la cascade de réacteurs.

2. procédé suivant la revendication 1, **caractérisé en ce que** l'on effectue la réaction à une température compris entre 50 et 90° C et sous une pression comprise entre 0,05 et 1 bar.

3. Procédé suivant l'une des revendications 1 à 2, **caractérisé en ce que** la durée de séjour totale dans les réacteurs est de 1 à 24 heures.

4. Procédé suivant l'une des revendications 1 à 3, **caractérisé en ce que** qu'il y a un excès d'urée par rapport au glyoxal dans le réacteur.

5. Procédé suivant l'une des revendications 1 à 4, **caractérisé en ce que**, pour la séparation mécanique de l'acétylènediurée de la liqueur mère, on utilise un groupe de filtration.

6. Procédé suivant la revendication 5, **caractérisé en ce que** l'on neutralise sur le groupe de filtration le gâteau de filtre obtenu en traitant par de la liqueur mère neutralisée.

7. Procédés suivant l'une des revendications 1 à 6, **caractérisé en ce que** l'on sèche l'acétylènediurée séparée dans un sécheur spinflash.
